# EUROPEAN PATENT APPLICATION

(11) **EP 2 314 325 A2**
(43) Date of publication of application: **27.04.2011**
(21) Application number: 10251698.6
(22) Date of filing: 30.09.2010
(51) Int. Cl.: A61L 24/00, A61L 24/04, A61L 24/08, A61L 24/10, A61L 27/20, A61L 27/22, A61L 27/26, A61L 27/54

(54) **Multi-mechanism surgical compositons**

(30) Priority: 01.10.2009 US 247707 P; 23.09.2010 US 888453
(71) Applicant: Tyco Healthcare Group LP, New Haven, CT 06511 (US)
(72) Inventor: Sargeant, Timothy, CT 06437 (US); Stopek, Joshua, CT 06437 (US)
(74) Representative: Soames, Candida Jane

(57) **Abstract**

The present disclosure provides hydrogel compositions having multiple gelation mechanisms. The composition includes at least one component which forms a hydrogel, in combination with a second component which includes a self-assembling peptide capable of forming a self-assembled macromer.

## Description

### CROSS-REFERENCE TO RELATED APPLICATIONS

This application claims the benefit of, and priority to, U.S. Provisional Patent Application Serial No. 61/247,707 filed on October 1, 2009, the disclosure of which is hereby incorporated by reference in its entirety.

### BACKGROUND

The present disclosure relates to compositions and methods for forming hydrogel compositions. In embodiments, the compositions include hydrogel compositions that are based on multiple gelation mechanisms.

Self-assembling peptides, such as, for example, peptide amphiphiles, may be used as bioactive mimics. These self-assembling peptides may be structured to mimic extracellular matrices or to surround and transport stem cells or other biologics. Self-assembling peptides may also be used to form implantable scaffolds for tissue growth. Certain amino acid residues used to form self-assembling peptides occur naturally within the body. This distinguishes self-assembling peptides from numerous other biocompatible substances and may offer unique advantages.

By contrast, hydrogels may be designed using specific polymerizable precursors and functional groups known to form consistent chemical structures. Hydrogels may be used in surgical applications for purposes such as, for example, drug delivery, as adhesives and/or sealants, and in forming implants.

Improved surgical compositions for use in drug delivery and as adhesives and/or sealants remain desirable.

### SUMMARY

The present disclosure is directed to multi-mechanism surgical compositions. In embodiments, a composition of the present disclosure may include at least one hydrogel precursor including functional groups such as electrophilic groups, nucleophilic groups, and combinations thereof; and at least one self-assembling peptide; wherein the hydrogel precursor forms a hydrogel composition concurrently with the self-assembling peptide forming a self-assembled macromer.

In embodiments, the present disclosure provides hydrogel compositions including at least one hydrogel precursor including electrophilic and nucleophilic functional groups; and at least one self-assembling peptide; wherein the hydrogel precursor forms a hydrogel composition prior to the self-assembling peptide forming a self-assembled macromer.

In other embodiments the present disclosure provides hydrogel compositions including at least one hydrogel precursor including electrophilic and nucleophilic functional groups; and at least one self-assembling peptide; wherein the self-assembling peptide forms a self-assembled macromer prior to the hydrogel precursor forming a hydrogel.

Implants formed from the compositions of the present disclosure are also provided herein. In embodiments, an implant may include at least one electrophilic polymer and at least one nucleophilic polymer; and, at least one self-assembling peptide; wherein the electrophilic polymer and the nucleophilic polymer form a composition prior to the self-assembling peptide forming a self-assembled macromer.

Methods for forming such compositions are also provided. In embodiments, methods of the present disclosure include providing at least one hydrogel precursor including functional groups such as electrophilic groups, nucleophilic groups, and combinations thereof; providing a self-assembling peptide; introducing the hydrogel precursor and the self-assembling peptide in situ; and initiating gelation of the self-assembling peptide and the hydrogel in situ.

### DETAILED DESCRIPTION

The present disclosure is directed to multi-mechanism surgical compositions. The multi-mechanism surgical composition includes at least two different components, which form by different mechanisms, optionally at different times. The separate components of the multi-mechanism surgical composition of the present disclosure include at least one hydrogel and at least one self-assembling peptide.

### Hydrogels

The first component of a composition of the present disclosure is a hydrogel. Hydrogels may be formed from synthetic and/or natural polymeric precursors. Natural hydrogel precursors, for example proteins, polysaccharides, or glycosaminoglycans, may be used as precursors to the hydrogel. Derivatives of proteins, polysaccharides, or glycosaminoglycans may also be used, such as, for example, hyaluronic acid, dextran, chondroitin sulfate, heparin, heparin sulfate, alginate, gelatin, collagen, albumin, ovalbumin, polyamino acids, collagen, fibrinogen, albumin, fibrin, starch, dermatan sulfate, keratan sulfate, dextran sulfate, pentosan polysulfate, chitosan, fibronectin, laminin, elastin, active peptide domains thereof, and combinations thereof.

As used herein, synthetic refers to a molecule not found in nature and does not include a derivatized version of a natural biomolecule, for example, collagen with modified side groups. Polyamino acid polymers generated synthetically are normally considered to be synthetic if they are not found in nature and are engineered to not be identical to naturally occurring biomolecules. Examples of synthetic precursors that may be used in accordance with the disclosure include, for example: polyethers, for example, polyalkylene oxides such as polyethylene glycol ("PEG"), polyethylene oxide ("PEO"), polyethylene oxide-co-polypropylene oxide ("PPO"), co-polyethylene oxide block or random copolymers; polyvinyl alcohol ("PVA"); poly (vinyl pyrrolidinone) ("PVP") as well as derivatives of the foregoing and combinations of the foregoing.

The hydrogel may include a single precursor or multiple precursors that form "in situ", meaning formation occurs at a tissue in a living animal or human body. In general, this may be accomplished by having a precursor that can be activated at the time of application to a tissue to form a hydrogel.

Hydrogels may be formed either through covalent, ionic or hydrophobic bonds. Physical (non-covalent) crosslinks may result from complexation, hydrogen bonding, desolvation, Van der Waals interactions, ionic bonding, combinations thereof, and the like, and may be initiated by mixing two precursors that are physically separated until combined in situ, or as a consequence of a prevalent condition in the physiological environment, including temperature, pH, ionic strength, combinations thereof, and the like. Chemical (covalent) crosslinking may be accomplished by any of a number of mechanisms, including free radical polymerization, condensation polymerization, anionic or cationic polymerization, step growth polymerization, electrophile-nucleophile reactions, combinations thereof, and the like.

In some embodiments, hydrogel systems may include biocompatible multi-precursor systems that spontaneously crosslink when the precursors are mixed, but wherein the two or more precursors are individually stable for the duration of the deposition process. Such systems include, for example for a hydrogel, a first precursor including macromers that are di- or multifunctional amines and a second precursor including di- or multifunctional ethylene oxide containing moieties.

Some embodiments of forming a hydrogel involve mixing precursors that crosslink quickly after application to a surface, e.g., on a tissue of a patient, to form a hydrogel.

The precursors may be placed into solution prior to use, with the solution being delivered to the patient. Solutions suitable for use in accordance with the present disclosure include those that may be used to form implants in lumens or voids. Where two solutions are employed, each solution may contain one precursor of a hydrogel which forms upon on contact. The solutions may be separately stored and mixed when delivered to tissue.

Additionally, any solutions utilized as part of the hydrogel system should not contain harmful or toxic solvents. In embodiments, the precursor(s) may be substantially soluble in a solvent such as water to allow application in a physiologically-compatible solution, such as buffered isotonic saline. Water-soluble coatings may form thin films, but in embodiments may also form three-dimensional gels of controlled thickness. The gel may also be biodegradable, so that it does not have to be retrieved from the body. Biodegradability, as used herein, refers to the predictable disintegration of the coating into molecules small enough to be metabolized or excreted under normal physiological conditions.

Properties of the hydrogel system may be selected according to the intended application. For example, if the hydrogel is to be used to temporarily occlude a reproductive organ, such as a fallopian tube, it may be desirable that the hydrogel system undergo significant swelling and be biodegradable. Alternatively, the hydrogel may have thrombotic properties, or its precursors may react with blood or other body fluids to form a coagulum.

Other applications may require different characteristics of the hydrogel system. Generally, the materials should be selected on the basis of exhibited biocompatibility and lack of toxicity.

Certain properties of the hydrogel can be useful, including adhesion to a variety of tissues, desirable setting times to enable a surgeon to accurately and conveniently place the hydrogels, high water content for biocompatibility, which may be relevant for hydrogels, mechanical strength for use in sealants, and/or toughness to resist destruction after placement. Synthetic materials that are readily sterilized and avoid the dangers of disease transmission involved in the use of natural materials may thus be used. Indeed, certain in situ polymerizable hydrogels made using synthetic precursors are within the purview of those skilled in the art, e.g., as used in commercially available products such as FOCALSEAL^{®} (Genzyme, Inc.), COSEAL^{®} (Angiotech Pharmaceuticals), and DURASEAL^{®} (Confluent Surgical, Inc). Other known hydrogels include, for example, those disclosed in U.S. Patent Nos. 6,656,200; 5,874,500; 5,543,441; 5,514,379; 5,410,016; 5,162,430; 5,324,775; 5,752,974; and 5,550,187.

As noted above, hydrogels may be made from one or more precursors. The precursor may be, e.g., a monomer or a macromer. One type of precursor may have a functional group that is ethylenically unsaturated. An ethylenically unsaturated functional group may be polymerized using an initiator to start the reaction. Precursors with at least two ethylenically unsaturated functional groups may form crosslinked polymers. Some compositions have certain precursors with only one such functional group and additional crosslinker precursors with a plurality of functional groups for crosslinking the precursors. Ethylenically unsaturated functional groups may be polymerized by various techniques, e.g., free radical, condensation, or addition polymerization. In embodiments, precursors possessing ethylenically unsaturated functional groups may be combined with an initiator to enhance crosslinking. Suitable initiators include, for example, thermal initiators, photoactivatable initiators, oxidation-reduction (redox) systems, and combinations thereof. In embodiments, sutiable initiators include those which, when exposed to ultraviolet light, enhance the crosslinking reaction.

Hydrogels may thus be formed from one precursor (as by free radical polymerization), two precursors, or made with three or more precursors, with one or more of the precursors participating in crosslinking to form the hydrogel.

Other precursors which may be used to form a hydrogel may have a functional group that is an electrophile or nucleophile. Electrophiles react with nucleophiles to form covalent bonds. Covalent crosslinks or bonds refer to chemical groups formed by reaction of functional groups on different polymers that serve to covalently bind the different polymers to each other. In certain embodiments, a first set of electrophilic functional groups on a first precursor may react with a second set of nucleophilic functional groups on a second precursor. When the precursors are mixed in an environment that permits reaction (e.g., as relating to pH or solvent), the functional groups react with each other to form covalent bonds. The precursors become crosslinked when at least some of the precursors can react with more than one other precursor. For instance, a precursor with two functional groups of a first type may be reacted with a crosslinking precursor that has at least three functional groups of a second type capable of reacting with the first type of functional groups.

As noted above, in embodiments functional groups may be electrophilic or nucleophilic groups that participate in an electrophilic-nucleophilic reaction to form a hydrogel. Examples of electrophilic functional groups include carbodiimidazole groups, sulfonyl chloride groups, chlorocarbonate groups, n-hydroxysuccinimidyl ester groups (NHS), succinimidyl ester groups, sulfosuccinimidyl ester groups, N-hydroxyethoxylated succinimide ester groups (ENHS), methane diisocyanate groups, methylene-bis(4-cyclohexylisocyanate) groups, isocyanate groups (NCO), cyanoacrylate, aldehyde, genipin, diisocyanate groups, hexamethylenediisocyanate groups, maleimide groups, combinations thereof, and the like.

Nucleophilic groups which may be present include, for example, -NH₂, -SH, -OH, -PH₂, and -CO-NH-NH₂, combinations thereof, and the like. Some non-limiting examples of nucleophilic polymers include collagen, gelatin, serum, and trilysine.

As mentioned above, in embodiments the hydrogel may be formed from single precursors or multiple precursors. For example, where the hydrogel is formed from multiple precursors, for example two precursors, the precursors may be referred to as a first and second hydrogel precursor. The terms "first hydrogel precursor" and "second hydrogel precursor" each mean a polymer, functional polymer, macromolecule, small molecule, or crosslinker that can take part in a reaction to form a network of crosslinked molecules, e.g., a hydrogel.

In embodiments, each of the first and second hydrogel precursors includes only one category of functional groups, either only nucleophilic groups or only electrophilic functional groups, so long as both nucleophilic and electrophilic precursors are used in the crosslinking reaction. Thus, for example, if the first hydrogel precursor has nucleophilic functional groups such as amines, the second hydrogel precursor may have electrophilic functional groups such as N-hydroxysuccinimides. On the other hand, if first hydrogel precursor has electrophilic functional groups such as sulfosuccinimides, then the second hydrogel precursor may have nucleophilic functional groups such as amines or thiols. Thus, functional polymers such as proteins, poly(allyl amine), styrene sulfonic acid, or amine-terminated di- or multifunctional poly(ethylene glycol) ("PEG") can be used.

The first and second hydrogel precursors may have biologically inert and water soluble cores. When the core is a polymeric region that is water soluble, suitable polymers that may be used include: polyethers, for example, polyalkylene oxides such as polyethylene glycol("PEG"), polyethylene oxide ("PEO"), polyethylene oxide-co-polypropylene oxide ("PPO"), co-polyethylene oxide block or random copolymers, and polyvinyl alcohol ("PVA"); poly(vinyl pyrrolidinone) ("PVP"); poly(amino acids); poly (saccharides), such as dextran, chitosan, alginates, carboxymethylcellulose, oxidized cellulose, hydroxyethylcellulose, hydroxymethylcellulose, hyaluronic acid, and proteins such as albumin, collagen, casein, and gelatin. The polyethers, and more particularly poly(oxyalkylenes) or poly(ethylene glycol) or polyethylene glycol, may be utilized in some embodiments. When the core is small in molecular nature, any of a variety of hydrophilic functionalities can be used to make the first and second hydrogel precursors water soluble. In embodiments, functional groups like hydroxyl, amine, sulfonate and carboxylate, which are water soluble, maybe used to make the precursor water soluble. For example, the, N-hydroxysuccinimide ("NHS") ester of subaric acid is insoluble in water, but by adding a sulfonate group to the succinimide ring, the NHS ester of subaric acid may be made water soluble, without affecting its reactivity towards amine groups.

In embodiments, at least one of the first and second hydrogel precursors is a crosslinker. In embodiments, at least one of the first and second hydrogel precursors is a macromolecule, and may be referred to herein as a "functional polymer".

Each of the first and second hydrogel precursors may be multifunctional, meaning that it may include two or more electrophilic or nucleophilic functional groups, such that, for example, a nucleophilic functional group on the first hydrogel precursor may react with an electrophilic functional group on the second hydrogel precursor to form a covalent bond. At least one of the first or second hydrogel precursors includes more than two functional groups, so that, as a result of electrophilic-nucleophilic reactions, the precursors combine to form cross-linked polymeric products.

In embodiments, a multifunctional nucleophilic polymer such as trilysine may be used as a first hydrogel precursor and a multifunctional electrophilic polymer such as a multi-arm PEG functionalized with multiple NHS groups may be used as a second hydrogel precursor. The multi-arm PEG functionalized with multiple NHS groups can, for example, have four, six or eight arms and a molecular weight of from about 2,000 Daltons (Da) to about 40,000 Da, in embodiments from about 10,000 Da to about 20,000 Da. Other examples of suitable first and second hydrogel precursors are described in U.S. Patent Nos. 6,152,943; 6,165,201; 6,179,862; 6,514,534; 6,566,406; 6,605,294; 6,673,093; 6,703,047; 6,818,018; 7,009,034; and 7,347,850, the entire disclosures of each of which are incorporated herein by reference.

In embodiments, one or more precursors having biodegradable linkages present in between functional groups may be included to make the hydrogel biodegradable or absorbable. In some embodiments, these linkages may be, for example, esters, which may be hydrolytically degraded in physiological solution. The use of such linkages is in contrast to protein linkages that may be degraded by proteolytic action. A biodegradable linkage optionally also may form part of a water soluble core of one or more of the precursors. Alternatively, or in addition, functional groups of precursors may be chosen such that the product of the reaction between them results in a biodegradable linkage. For each approach, biodegradable linkages may be chosen such that the resulting biodegradable biocompatible crosslinked polymer degrades or is absorbed in a desired period of time. Generally, biodegradable linkages may be selected that degrade the hydrogel under physiological conditions into non-toxic or low toxicity products.

In embodiments a hydrogel may also include an initiator. An initiator may be any precursor or group capable of initiating a polymerization reaction for the formation of the hydrogel.

The reaction conditions for forming crosslinked polymeric hydrogels will depend on the nature of the functional groups. In embodiments, reactions are conducted in buffered aqueous solutions at a pH of about 5 to about 12. Buffers include, for example, sodium borate buffer (pH 10) and triethanol amine buffer (pH 7). In some embodiments, organic solvents such as ethanol or isopropanol may be added to improve the reaction speed or to adjust the viscosity of a given formulation.

When the crosslinker and functional polymers are synthetic (for example, when they are based on polyalkylene oxide), it may be desirable to use molar equivalent quantities of the reactants. In some cases, molar excess of a crosslinker may be added to compensate for side reactions such as reactions due to hydrolysis of the functional group.

Synthetic crosslinked gels degrade due to hydrolysis of the biodegradable region. The degradation of gels containing synthetic peptide sequences will depend on the specific enzyme and its concentration. In some cases, a specific enzyme may be added during the crosslinking reaction to accelerate the degradation process.

When choosing the crosslinker and crosslinkable polymer, at least one of the polymers may have more than two functional groups per molecule and at least one degradable region, if it is desired that the resultant biocompatible crosslinked polymer be biodegradable. In embodiments, each biocompatible crosslinked polymer precursor may have more than two functional groups, and in some embodiments, more than four functional groups.

The crosslinking density of the resultant biocompatible crosslinked polymer may be controlled by the overall molecular weight of the crosslinker and functional polymer and the number of functional groups available per molecule. A lower molecular weight between crosslinks, such as 600 Da, will give much higher crosslinking density as compared to a higher molecular weight, such as 10,000 Da. Elastic gels may be obtained with higher molecular weight functional polymers with molecular weights of more than 3000 Da.

The crosslinking density may also be controlled by the overall percent solids of the crosslinker and functional polymer solutions. Increasing the percent solids increases the probability that an electrophilic group will combine with a nucleophilic group prior to inactivation by hydrolysis. Yet another method to control crosslink density is by adjusting the stoichiometry of nucleophilic groups to electrophilic groups. A one to one ratio may lead to the highest crosslink density, however, other ratios of reactive functional groups (e.g., electrophile:nucleophile) are envisioned to suit a desired formulation.

Biodegradable crosslinkers or small molecules as described above may be reacted with proteins, such as albumin, other serum proteins, or serum concentrates to generate crosslinked polymeric networks. Generally, aqueous solutions of crosslinkers may be mixed with concentrated solutions of proteins to produce a crosslinked hydrogel. The reaction may be accelerated by adding a buffering agent, e.g., borate buffer or triethanol amine, during the crosslinking step.

The resulting crosslinked hydrogel's degradation depends on the degradable segment in the crosslinker as well as degradation by enzymes. In the absence of any degradable enzymes, the crosslinked polymer may degrade solely by hydrolysis of the biodegradable segment. In embodiments in which polyglycolate is used as the biodegradable segment, the crosslinked polymer may degrade in from about 1 day to about 30 days depending on the crosslinking density of the network. Similarly, in embodiments in which a polycaprolactone based crosslinked network is used, degradation may occur over a period of from about 1 month to about 8 months. The degradation time generally varies according to the type of degradable segment used, in the following order: polyglycolate<polylactate<polytrimethylene carbonate<polycaprolactone. Thus, it is possible to construct a hydrogel with a desired degradation profile, from a few days to months, using a proper degradable segment.

The hydrophobicity generated by biodegradable blocks such as oligohydroxy acid blocks or the hydrophobicity of PPO blocks in PLURONIC or TETRONIC polymers are helpful in dissolving small organic drug molecules. Other properties which will be affected by incorporation of biodegradable or hydrophobic blocks include: water absorption, mechanical properties and thermosensitivity.

In some embodiments, formulations may be prepared that are suited to make precursor crosslinking reactions occur in situ. In general, this may be accomplished by having a precursor that can be activated at the time of application to a tissue to form a crosslinked hydrogel. Activation can be made before, during, or after application of the precursor to the tissue, provided that the precursor is allowed to conform to the tissue's shape before crosslinking and associated gelation is otherwise too far advanced. Activation includes, for instance, triggering a polymerization process, initiating a free radical polymerization, or mixing precursors with functional groups that react with each other. Thus, in situ polymerization includes activation of chemical moieties to form covalent bonds to create an insoluble material, e.g., a hydrogel, at a location where the material is to be placed on, within, or both on and within, a patient. In situ polymerizable polymers may be prepared from precursors that can be reacted such that they form a polymer within the patient. Thus precursors with electrophilic functional groups can be mixed or otherwise activated in the presence of precursors with nucleophilic functional groups. In other embodiments, precursors with ethylenically unsaturated groups can be initiated to polymerize in situ on the tissue of a patient. In yet other embodiments, the precursors may form a hydrogel in the form of a film, the film can later be implanted in situ. In alternate embodiments, a hydrogel may be lyophilized to create a foam, which can be later implanted in situ.

With respect to coating a tissue, and without limiting the present disclosure to a particular theory of operation, it is believed that reactive precursor species that crosslink quickly after contacting a tissue surface may form a three dimensional structure that is mechanically interlocked with the coated tissue. This interlocking contributes to adherence, intimate contact, and continuous coverage of the coated region of the tissue.

In embodiments, a precursor may include functional groups capable of reacting with amines present in the tissue to which the polymeric precursor is applied.

### Visualization Agents

The precursor and/or the crosslinked polymer may contain visualization agents to improve their visibility during surgical procedures. Visualization agents may be selected from a variety of non-toxic colored substances, such as dyes, suitable for use in implantable medical devices. Suitable dyes are within the purview of those skilled in the art and may include, for example, a dye for visualizing a thickness of the hydrogel as it is formed in situ, e.g., as described in U.S. Patent No. 7,009,034. In some embodiments, a suitable dye may include, for example, FD&C Blue #1, FD&C Blue #2, FD&C Blue #3, FD&C Blue #6, D&C Green #6, methylene blue, indocyanine green, other colored dyes, and combinations thereof. It is envisioned that additional visualization agents may be used such as fluorescent compounds (e.g., flurescein or eosin), x-ray contrast agents (e.g., iodinated compounds), ultrasonic contrast agents, and MRI contrast agents (e.g., Gadolinium containing compounds).

The visualization agent may be present in either a crosslinker or functional polymer solution. The colored substance may or may not become incorporated into the biocompatible crosslinked polymer. In embodiments, however, the visualization agent does not have a functional group capable of reacting with the crosslinker or functional polymer.

The visualization agent may be used in small quantities, in embodiments less than 1% weight/volume, and in other embodiments less that 0.01% weight/volume and in yet other embodiments less than 0.001 % weight/volume concentration.

### Self-Assembling Peptides

The second component of the compositions of the present disclosure includes self-assembling peptides that form a macromer on their own. The term "peptide," as used herein includes "polypeptide," "oligopeptide," and "protein," and refers to a string of at least two α-amino acid residues linked together by chemical bonds (for example, peptide bonds). Depending on the context, "peptide" may refer to an individual peptide or to a collection of peptides having the same or different sequences, any of which may contain only naturally occurring α-amino acid residues, non-naturally occurring α-amino acid residues, or both. Self-assembling peptides include, for example, peptide amphiphiles, and peptides with (β-sheet or α-helical forming sequences.

The peptides may include D-amino acids, L-amino acids, or combinations thereof. Suitable, naturally-occurring hydrophobic amino acid residues which may be in the self-assembling peptides include Ala, Val, Ile, Met, Phe, Tyr, Trp, Ser, Thr and Gly. The hydrophilic amino acid residues may be basic amino acids (for example, Lys, Arg, His, Orn); acidic amino acids (for example, Glu, Asp); or amino acids that form hydrogen bonds (for example, Asn, Gln). Degradation of L-amino acids produces amino acids that may be reused by the host tissue. L-configured amino acid residues occur naturally within the body, distinguishing peptides formed from this class of compounds from numerous other biocompatible substances. L-configured amino acids contain biologically active sequences such as RGD adhesion sequences.

The amino acid residues in the self-assembling peptides may be naturally occurring or non-naturally occurring amino acid residues. Naturally occurring amino acids may include amino acid residues encoded by the standard genetic code, amino acids that may be formed by modifications of standard amino acids (for example pyrrolysine or selenocysteine), as well as non-standard amino acids (for example, amino acids having the D-configuration instead of the L-configuration). Although, non-naturally occurring amino acids have not been found in nature, they may be incorporated into a peptide chain. These include, for example, D-alloisoleucine(2R,3S)-2-amino-3-methylpentanoic acid, L-cyclopentyl glycine (S)-2-amino-2-cyclopentyl acetic acid.

Self-assembling peptides used in accordance with the disclosure may vary in length so long as they retain the ability to self-assemble to an extent useful for one or more of the purposes described herein. Peptides having as few as two a-amino acid residues or as many as approximately 30 residues may be suitable. In embodiments, a-amino acid analogs can be used. In particular, a-amino acid residues of the D-form may be used. Useful peptides may also be branched.

In embodiments, one or more of the amino acid residues in a self-assembling peptide may be functionalized by the addition of a chemical entity such as an acyl group, a carbohydrate group, a phosphate group, a farnesyl group, an isofarnesyl group, a fatty acid group, or a linker for conjugation. This functional group may provide for inter-peptide linkages, or linkages between the peptide and the hydrogel or hydrogel precursor. For example, the hydrophobic portion of an amphiphilic peptide may be functionalized with acetylene groups. Alternatively, hyaluronic acid (HA) may be reacted with amphiphilic peptides on a supramolecular level.

Either or both ends of a given peptide may be modified. For example, the carboxyl and/or amino groups of the carboxyl- and amino-terminal residues, respectively, may be protected or not protected. The charge at a terminus may also be modified. For example, a group or radical such as an acyl group (RCO--, where R is an organic group (for example, an acetyl group (CH₃C0--)) may be present at the N-terminus of a peptide to neutralize an "extra" positive charge that may otherwise be present (for example, a charge not resulting from the side chain of the N-terminal amino acid). Similarly, a group such as an amine group (NH₂) can be used to neutralize an "extra" negative charge that may otherwise be present at the C-terminus (for example, a charge not resulting from the side chain of the C-terminal amino acid residue). Where an amine is used, the C-terminus may have an amide (--CONH₂). The neutralization of charges on a terminus may facilitate self-assembly. One of ordinary skill in the art could select other suitable groups.

In embodiments, the self-assembling peptides may be therapeutic peptides. Therapeutic peptides include, for example, vasopressin analogues, GNRH/LHRH agonists such as leuprorelin or goserelin, somatostatin analogues, immunomodulator peptides, calcitonins and platelet aggregate inhibitors.

As noted above, in embodiments the self-assembling peptides may be amphiphilic peptides. Amphiphilic peptides may have varying sequences, which convey an amphiphilic nature to the peptides (for example, the peptides can include approximately equal numbers of hydrophobic and hydrophilic amino acid residues). The amphiphilic peptide may include at least one portion which is hydrophilic and at least one portion which is hydrophobic.

Some examples of self-assembling peptides that may be utilized in accordance with the present disclosure include, for example, EAK16-II (commercially available from AnaSpec, Inc.); the islet amyloid polypeptide hIAPP(22-27) (commercially available from AnaSpec, Inc.); RAD16-I, combinations thereof, and the like. Other self-assembling peptides include those disclosed by Nagai, et al. "Slow Release of Molecules in Self-Assembling Peptide Nanofiber Scaffold", Journal of Controlled Release Vol. 115, pp. 18-25 (2006); Schneider, et al. "Self-Assembling Peptide Nanofiber Scaffolds Accelerate Wound Healing", PLoS ONE, Issue 1, pp. 1-8 (2008); Hartgerink, et al. "Peptide-Amphiphile Nanofibers: A Versatile Scaffold for the Preparation of Self-Assembling Materials", PNAS Early Edition, pp. 1-6 (2001); and Silva, et al. "Selective Differentiation of Neural Progenitor Cells by High-Epitope Density Nanofibers", Science, Vol. 303, pp. 1352-1355 (2004), the entire disclosures of each of which are incorporated by reference herein.

Without being limited, it is believed the side-chains (or R groups) of self-assembling peptides partition into two faces, a polar face with positively and/or negatively charged ionic side chains, and a nonpolar face with side chains that are considered neutral or uncharged at physiological pH (for example, the side chain of an alanine residue or residues having other hydrophobic groups). The positively charged and negatively charged amino acid residues on the polar face of one peptide can form complementary ionic pairs with oppositely charged residues of another peptide and are thus capable of self-assembly. These peptides may therefore be called ionic self-assembling peptides.

If the ionic residues alternate with one positively and one negatively charged residue on the polar face (-+-+-+-+), the peptides may be described as "modulus I" ; if the ionic residues alternate with two positively and two negatively charged residues (--++--++) on the polar face, the peptides may be described as "modulus II" ; if the ionic residues alternate with three positively and three negatively charged residues (+++---+++---) on the polar face, the peptides may be described as "modulus III" ; if the ionic residues alternate with four positively and four negatively charged residues (++++----++++----) on the polar face, they may be described as "modulus IV." Positively charged residues in these peptides (lysine and arginine) may be replaced by negatively charged residues (aspartate and glutamate) to form a beta-sheet macromer structure in the presence of a salt.

Additional factors important for the formation of macromer structures from self-assembling peptides include, for example, peptide length, the degree of intermolecular interaction, and the ability to form staggered arrays. Peptide-based structures may be formed of heterogeneous mixtures of peptides (i.e., mixtures containing more than one type of peptide conforming to a given modulus or to two or more of the above modulus). In embodiments, each type of peptide may not, alone, self-assemble but the combination of heterogeneous peptides may self-assemble (i.e., peptides in the mixture are complementary and structurally compatible with each other). In other embodiments, each of the types of peptides in the mixture is able to self-assemble by itself. Thus, either a homogeneous mixture of self-complementary and self-compatible peptides of the same sequence, or containing the same repeating subunit, or a heterogeneous mixture of different peptides which are complementary and structurally compatible to each other, may be used.

The selection of amino acids (on the self-assembling peptides) can be used to influence secondary structure such as beta sheet formation. Some self-assembling peptides may form alpha-helices and random-coils rather than beta-sheet macromers.

Two or more peptides may also be linked by an interaction such as, for example, chiral dipole-dipole interactions, π-π stacking, hydrogen bonds, van der Waals interactions, hydrophobic forces, electrostatic interactions and/or repulsive steric forces.

Other useful self-assembling peptides may be generated, for example, which differ from those exemplified by a single amino acid residue or by multiple amino acid residues (for example, by inclusion or exclusion of a repeating quartet). For example, one or more cysteine residues may be incorporated into the peptides, and these residues may bond with one another by the formation of disulfide bonds. Structures bonded in this manner may have increased mechanical strength relative to structures made with comparable peptides that do not include cysteine residues.

Self-assembling peptides may be chemically synthesized or purified from natural or recombinantly-produced sources by methods within the purview of those skilled in the art. For example, peptides can be synthesized using standard f-moc chemistry and purified using high pressure liquid chromatography (HPLC). F-moc chemistry involves the protection of an amino acid side chain with 9H-fluoren-9-ylmethyoxycarbonyl (f-moc). A second protected amino acid is attached to a resin. Protected amino acids are exposed to the resin bound amino acid and the f-moc is removed by a mildly basic solution such as piperidine thereby allowing the amino acids to bond. In this manner, a specific sequence of amino acids may be used to form a peptide. When the desired sequence is formed, the resulting peptide is cleaved from the resin.

In embodiments, the hydrogel, the self-assembling peptides, or both, may be combined with solvents for application. Solvents which may be utilized include biocompatible solvents. For amphiphilic materials, the solvent should be a good solvent for one portion of the peptide and a poor solvent for the other portion of the peptide. When added to a hydrophilic solvent, the peptides self-assemble to form a micelle, with the hydrophilic portions aligned along the exterior of the micelle and the hydrophobic portions gathered near the interior of the micelle. Under certain conditions, the micelle formed is a linear micelle or nanofiber macromer. Any peptides which have been functionalized may react to provide stability to the self assembled macromer structure. Where the self assembled structure is a linear micelle or nanofiber macromer, the functional groups may provide radial cross-linking as well as longitudinal cross-linking along the length of the linear micelle or nanofiber macromer.

Suitable solvents include aqueous solvents such as water, saline, salt buffers and cell medium. It is also envisioned that other polar and non-polar solvents may be employed if they do not interfere with the self-assembly mechanism of the peptides.

The product of the self-assembling peptides, sometimes referred to herein, in embodiments, as a self-assembled macromer, ma y be formed that have varying degrees of stiffness or elasticity. The resulting macromer structures may have a low elastic modulus (for example, a modulus in the range of 1-10 kPa as measured by standard methods, such as in a standard cone-plate rheometer). Low values may be desirable, as they permit structure deformation as a result of movement, in response to pressure, and/or in the event of cell contraction. More specifically, stiffness may be controlled in a variety of ways, including by changing the length, sequence, and/or concentration of the precursor molecules (for example, self-assembling peptides). Other methods for increasing stiffness may also be employed.

The extent of cross-linking of a macromer formed by self-assembling peptides may be determined by light scattering, gel filtration, or scanning electron microscopy using standard methods. Furthermore, cross-linking may be examined by HPLC or mass spectrometry analysis of the structure after digestion with a protease, such as matrix metalloproteases. Material strength may be determined before and after cross-linking.

The half-life (for example, the in vivo half-life) of the macromer structures formed by self-assembling peptides may also be modulated by incorporating protease or peptidase cleavage sites into the precursors that subsequently form the macromer structure. Proteases or peptidases that occur naturally in vivo or that are introduced (for example, during application) may then promote degradation. Combinations of the modifications described herein may be made. For example, self-assembling peptides that include a protease cleavage site and a cysteine residue and/or a cross-linking agent, kits and devices containing them, and methods of using them can be utilized.

In embodiments, macromer formation by a self-assembling peptide, may be initiated by, for example, UV light, or ions. Long wave UV and visible light photoactivatable initiators include, for example, ethyl eosin groups, 2,2-dimethoxy-2-phenyl acetophenone groups, other acetophenone derivatives, thioxanthone groups, benzophenone groups, camphorquinone groups, combinations thereof, and the like.

A wide variety of ions, including anions and cations (whether divalent, monovalent, or trivalent) may be used to initiate self-assembly of peptides. For example, one may initiate self assembly by exposure to divalent ions such as Ca ²⁺ , Mg ²⁺ and the like, and the concentration of such ions required to induce or enhance self-assembly may be at least 5 mM (for example, at least 10, 20, or 50 mM). Lower concentrations also facilitate assembly, though at a reduced rate. When desired, self-assembling peptides can be delivered with a hydrophobic material (for example a pharmaceutically acceptable oil) in a concentration that permits self-assembly, but at a reduced rate.

### Multi-Mechanism Composition Formation

The hydrogel and the self-assembling peptide of the disclosure may be induced to form concurrently or separately. As used herein, "concurrently" means that hydrogel and self-assembling peptide formation begins to occur at exactly or nearly exactly the same time. In other embodiments, the hydrogel is formed first to create a structure into which the self-assembling peptide may align during macromer formation. In other embodiments, the self-assembling peptide is formed first in order to insure homogeneous formation after which the hydrogel is formed. The hydrogel and the self-assembled peptide may be: physically entangled; a series of interpenetrating networks; layered; woven together; the hydrogel may surround the self-assembled macromer; the self-assembled macromer may surround the hydrogel; combinations thereof, and the like. It is also conceived herein that the hydrogel and the self-assembled macromer may interact through the same mechanisms that peptides of the self-assembled macromer interact, i.e., chemically through the formation of bonds, such as, for example, ionic bonds, covalent bonds, chiral dipole-dipole interactions, hydrogen bonds, van der Waals interactions, hydrophobic forces, π-π stacking electrostatic interactions, or repulsive steric forces.

In embodiments the multi-mechanism hydrogel is porous following formation. In other embodiments, the multi-mechanism hydrogel is smooth or non-porous following formation; smooth hydrogels may be formed, in embodiments, by dehydrating a formed hydrogel. Either or both of the hydrogel and the self-assembled macromer may be non-absorbable or absorbable. In some embodiments, both the hydrogel and the self-assembled macromer are absorbable. Alternatively, the hydrogel may be non-absorbable and the self-assembled macromer may be absorbable, or vice versa. It is also envisioned that each of the hydrogel and the self-assembled macromer may include both absorbable and non-absorbable components.

Formulations of the hydrogel and/or the self-assembled macromer may gel in situ or prior to implantation. In situ formation may be accomplished by having hydrogel precursor(s) that can be activated at the time of application to tissue to form a cross-linked hydrogel. For in situ formation, initiation of cross-linking can be made before, during, or after application of the precursor(s) to the tissue, provided that the precursor(s) is allowed to conform to the tissue's shape before cross-linking and associated formation is otherwise too far advanced.

The cross-linking reaction leading to formation of the hydrogel can occur, in some embodiments within a time from about 1 second to about 5 minutes, in embodiments from about 3 seconds to about 1 minute; persons of ordinary skill in these arts will immediately appreciate that all ranges and values within these explicitly stated ranges are contemplated. In some cases formation may occur in less than 10 seconds.

In embodiments, the self-assembling peptides may form the self-assembled macromers when the compositions are exposed to or brought into contact with body tissue. Self-assembly following tissue contact may occur, for example, after more than about 1 second.

In embodiments, the formation of the hydrogel and the self-assembled macromer may occur concurrently. In embodiments, the cross-linking of the hydrogel precursors occurs prior to the formation of the self-assembled macromer. In embodiments, the hydrogel forms in less than one minute. The formed hydrogel may then serve as a scaffold for homogeneous distribution of the self-assembling peptides, which, in embodiments, form macromers in a time period greater than one minute. The staggered formation of the hydrogel and the self assembled macromer may occur in situ or prior to implantation. In yet other embodiments, the self-assembling peptides may form the self-assembled macromer prior to the formation of the hydrogel. For example, in embodiments, the self-assembled macromer may be formed immediately after contact with tissue, with the hydrogel formed from about 5 seconds to about 5 minutes after contact with tissue.

Optional initiators for the hydrogel and for the self-assembled macromer may be the same or different. In embodiments, where the initiator group for both the hydrogel and the self-assembled macromer is the same, the initiator group may be, for example, UV light, or ions.

Compositions of the present disclosure may include the hydrogel in an amount of from about 1.5 to about 25 percent by weight of the compositions, in embodiments from about 3 to about 15 percent by weight of the compositions, with the self-assembled macromer present in an amount of from about 0.5 to about 10 percent by weight of the compositions, in embodiments from about 1 to about 3 percent by weight of the compositions.

### Additives

The multi-mechanism hydrogel of the present disclosure may include at least one bioactive agent. In some embodiments the hydrogel and/or the self-assembled macromer may possess at least one bioactive agent. The term "bioactive agent", as used herein, is used in its broadest sense and includes any substance or mixture of substances that have clinical use. Consequently, bioactive agents may or may not have pharmacological activity per se, for example, a dye. Alternatively, a bioactive agent could be any agent which provides a therapeutic or prophylactic effect; a compound that affects or participates in tissue growth, cell growth and/or cell differentiation; a compound that may be able to invoke a biological action such as an immune response; or a compound that could play any other role in one or more biological processes.

Examples of classes of bioactive agents which may be utilized in accordance with the present disclosure include antimicrobials, analgesics, antipyretics, antiadhesive agents, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, narcotics, lipids, lipopolysaccharides, polysaccharides, peptides, proteins, hormones and enzymes. It is also intended that combinations of bioactive agents may be used.

Suitable antimicrobial agents which may be included as a bioactive agent in the of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent.

Other bioactive agents which may be included as a bioactive agent include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (for example oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anticancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included in the hydrogel and/or the self-assembled macromer include viruses and cells, peptides, polypeptides and proteins, analogs, muteins, and active fragments thereof, such as immunoglobulins, antibodies, cytokines (for example lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (for example, GCSF, GM-CSF, MCSF), insulin, anti-tumor agents and tumor suppressors, blood proteins, gonadotropins (for example, FSH, LH, CG, etc.), hormones and hormone analogs (for example, growth hormone), vaccines (for example, tumoral, bacterial and viral antigens); somatostatin; antigens; blood coagulation factors; growth factors (for example, nerve growth factor, insulin-like growth factor); protein inhibitors, protein antagonists, and protein agonists; nucleic acids, such as antisense molecules, DNA and RNA; oligonucleotides; and ribozymes.

In embodiments, a single bioactive agent may be utilized in the multi-mechanism composition of the present disclosure or, in alternate embodiments, any combination of bioactive agents may be utilized in the multi-mechanism composition of the present disclosure.

Absorbable materials which may be combined with a bioactive agent and utilized in the multi-mechanism composition include soluble hydrogels such as gelatin or a starch, or cellulose-based hydrogels. In embodiments, the absorbable material may be an alginate or hyaluronic acid. Other examples of absorbable materials which may be utilized in the multi-mechanism composition include trimethylene carbonate, caprolactone, dioxanone, glycolic acid, lactic acid, glycolide, lactide, homopolymers thereof, copolymers thereof, and combinations thereof.

### Administration

The multi-mechanism surgical composition may be placed into solution prior to use, with the solution being delivered to the patient. The hydrogel system solutions should not contain harmful or toxic solvents. In embodiments, the multi-mechanism composition may be substantially soluble in water to allow application in a physiologically-compatible solution, such as buffered isotonic saline. One may use a dual syringe or similar device to apply the precursor solutions, such as those described in U.S. Patent Nos. 4,874,368; 4,631,055; 4,735,616; 4,359,049; 4,978,336; 5,116,315; 4,902,281; 4,932,942; 6,179,862; 6,673,093; and 6,152,943.

### Applications

The multi-mechanism composition may be used as a substitute for, or in conjunction with, known hydrogel compositions. For example, the multi-mechanism composition may be used as a tissue adhesive, to adhere implants to tissue in situ, for reformation of tissue in situ, to form sustained release delivery of the bioactive agents listed above, and other uses within the purview of those skilled in the art.

The multi-mechanism composition may be used in both open and minimally invasive surgical procedures. For example, the multi-mechanism composition may be used during laparoscopic, endoscopic or arthroscopic surgery.

In order that those skilled in the art may be better able to practice the features of the present disclosure described herein, the following examples are provided to illustrate, but not limit, the features of the present disclosure.

### EXAMPLE 1

A collagen solution is combined with pre-formed self assembling peptides nanofibers. The self assembling peptides are either all negatively charged or a mixture of positively and negatively charged. The collagen solution is then sprayed in situ using one syringe, while a second syringe containing the electrophilic functional group is simultaneously sprayed.

### EXAMPLE 2

A nucleophilic functional polymer solution and an electrophilic functional polymer solution are simultaneously sprayed in situ. The self assembling peptides are contained within the either the nucleophilic or electrophilic solution such that they are soluble. Once mixed in situ, the peptides self assemble to create a peptide macromer while the electrophilic and nucleophilic polymer react, to form a crosslinked gel.

### EXAMPLE 3

Nucleophilic and electrophilic precursors are simultaneously sprayed into a mold to create a hydrogel of set dimensions. The hydrogel is then lyophilized to create a foam. Prior to implanting in situ, the foam is immersed in a self-assembling peptide solution (aqueous). Once implanted, the peptides begin to self assemble, creating a peptide macromer.

While the above description contains many specifics, these specifics should not be construed as limitations on the scope of the present disclosure, but merely as exemplifications of preferred embodiments thereof. Those skilled in the art will envision many other possible variations that are within the scope and spirit of the present disclosure.

## Claims

1. A composition comprising:
at least one hydrogel precursor comprising functional groups selected from the group consisting of electrophilic groups, nucleophilic groups, and combinations thereof; and
at least one self-assembling peptide;
wherein the hydrogel precursor forms a hydrogel composition concurrently with the self-assembling peptide forming a self-assembled macromer.

2. The composition of claim 1, wherein the self-assembling peptide is an amphiphilic peptide.

3. The composition of claim 1 or claim 2, wherein the electrophilic functional groups are selected from the group consisting of carbodiimidazole groups, sulfonyl chloride groups, chlorocarbonate groups, n-hydroxysuccinimidyl ester groups, succinimidyl ester groups, sulfosuccinimidyl ester groups, N-hydroxyethoxylated succinimide ester groups, methane diisocyanate groups, methylene-bis(4-cyclohexylisocyanate) groups, isocyanate groups, cyanoacrylates, aldehydes, genipin, diisocyanate groups, hexamethylenediisocyanate groups, maleimide groups, and combinations thereof and/or wherein the nucleophilic functional group is selected from the group consisting -NH₂, -SH, -OH, -PH₂, and -CO-NH-NH₂, and combinations thereof.

4. The composition of any preceding claim, wherein the at least one hydrogel precursor comprises a first hydrogel precursor comprising electrophilic groups in combination with a second hydrogel precursor comprising nucleophilic groups, preferably wherein the electrophilic functional group of the first hydrogel precursor comprises an n-hydroxysuccinimdyl ester and wherein the second hydrogel precursor comprises trilysine.

5. The composition of any preceding claim, wherein the composition further comprises a bioactive agent.

6. A hydrogel composition comprising:
at least one hydrogel precursor comprising electrophilic and nucleophilic functional groups; and
at least one self-assembling peptide;
wherein the hydrogel precursor forms a hydrogel composition prior to the self-assembling peptide forming a self-assembled macromer.

7. An implant comprising:
at least one electrophilic polymer and at least one nucleophilic polymer; and,
at least one self-assembling peptide;
wherein the electrophilic polymer and the nucleophilic polymer form a composition prior to the self-assembling peptide forming a self-assembled macromer.

8. The implant according to claim 7, wherein the composition is selected from the group consisting of films, foams, tissue scaffolds, and drug delivery devices.

9. The implant according to claim 8, wherein the composition at least partially hydrated in a peptide solution, preferably wherein the self-assembling peptide is combined with an aqueous solution.

10. A hydrogel composition comprising:
at least one hydrogel precursor comprising electrophilic and nucleophilic functional groups; and
at least one self-assembling peptide;
wherein the self-assembling peptide forms a self-assembled macromer prior to the hydrogel precursor forming a hydrogel.

11. The hydrogel composition of claim 10, wherein the hydrogel forms a hydrogel composition in from about 5 seconds to about 5 minutes.

12. The hydrogel composition of claim 10 or claim 11, wherein the nucleophilic functional group comprises a polymer selected from the group consisting of collagen, gelatin, or serum, preferably wherein the nucleophilic functional group further comprises the at least one self-assembling peptide.

13. A method for forming a composition in situ comprising:
providing at least one hydrogel precursor comprising functional groups selected from the group consisting of electrophilic groups, nucleophilic groups, and combinations thereof;
providing a self-assembling peptide;
introducing the hydrogel precursor and the self-assembling peptide in situ; and
initiating gelation of the self-assembling peptide and the hydrogel in situ.

14. The method of claim 13, wherein the gelation of the self-assembling peptide and the hydrogel precursor occurs concurrently, or the method of claim 13, wherein the gelation of the self-assembling peptide occurs prior to gelation of the hydrogel precursor, or the method of claim 13, wherein the gelation of the self-assembling peptide occurs after gelation of the hydrogel precursor.

15. The method of claim 13 or claim 14, wherein the electrophilic functional groups are selected from the group consisting of carbodiimidazole groups, sulfonyl chloride groups, chlorocarbonate groups, n-hydroxysuccinimidyl ester groups, succinimidyl ester groups, sulfosuccinimidyl ester groups, N-hydroxyethoxylated succinimide ester groups, methane diisocyanate groups, methylene-bis(4-cyclohexylisocyanate) groups, isocyanate groups, cyanoacrylates, aldehydes, genipin, diisocyanate groups, hexamethylenediisocyanate groups, maleimide groups, and combinations therof; and/or the method of claim 13 or claim 14, wherein the nucleophilic functional group is selected from the group consisting -NH₂, -SH, -OH, -PH₂, and -CO-NH-NH₂, and combinations thereof.

16. The method of claim 15, wherein the at least one hydrogel precursor comprises a first hydrogel precursor comprising electrophilic groups in combination with a second hydrogel precursor comprising nucleophilic groups.

17. The method of any of claims 13 to 16, wherein the electrophilic functional group of the first hydrogel precursor comprises an n-hydroxysuccinimdyl ester and wherein the second hydrogel precursor comprises a polymer selected from the group consisting of trilysine, collagen, gelatin, and serum.
